# EUROPEAN PATENT SPECIFICATION

(11) **EP 4 029 552 B1**
(45) Date of publication and mention of the grant of the patent: **16.04.2025**
(21) Application number: 20862748.9
(22) Date of filing: 08.09.2020
(51) Int. Cl.: A61M 16/00, G10K 11/16, F16L 55/02

(54) **NOISE REDUCTION BOX AND VENTILATION THERAPEUTIC DEVICE**
SCHALLSCHLUCKBOX UND THERAPEUTISCHE VENTILATIONSVORRICHTUNG
BOÎTE DE RÉDUCTION DE BRUIT ET DISPOSITIF THÉRAPEUTIQUE DE VENTILATION

(30) Priority: 09.09.2019 CN 201910849358
(43) Date of publication of application: 20.07.2022
(73) Proprietor: BMC Medical Co., Ltd., Shijingshan Beijing 100041 (CN)
(72) Inventor: ZHI, Jianxin, Beijing 100041 (CN); ZHUANG, Zhi, Beijing 100041 (CN)
(74) Representative: Grünecker Patent- und Rechtsanwälte PartG mbB
(86) International application number: PCT/CN2020/113861
(87) International publication number: WO 2021/047486

(56) References cited:
- CN-A- 101 171 048
- CN-A- 101 850 146
- CN-A- 101 850 146
- CN-A- 104 132 003
- CN-A- 104 180 444
- CN-A- 110 711 300
- CN-U- 205 689 989
- CN-U- 206 637 817
- CN-U- 209 265 670
- CN-U- 211 132 546
- CN-U- 211 884 847
- US-A1- 2014 299 130

## Description

### FIELD

The present disclosure relates to the field of ventilation therapeutic devices, in particular to a noise reduction box comprising a shell and a detection assembly, wherein the inside of the shell defines a cavity and the shell is provided with an air inlet and an air outlet that are in communication with the cavity and wherein the detection assembly is mounted on the shell, and a ventilation therapeutic device including the same.

### BACKGROUND

In ventilation therapeutic devices, such as ventilators, the noise reduction box is a very important component for mounting a fan and reducing the noise of the fan, so that the fan can draw external breathable gas (e.g., air) and then transfer the external breathable gas to a humidifier of the ventilator or directly deliver the external breathable gas to the service side, such as a breathing mask. However, owing to the fact that the air usually contains pollutants such as dust, the inside of the noise reduction box will become dirty to a degree as the ventilator is used, resulting in a hygienic problem. The human body health may be endangered if the inside of the noise reduction box is dirty to a high degree. Therefore, it is necessary to detect the degree to which the inside of the noise reduction box is dirty, so that the noise reduction box can be cleaned and serviced timely.

However, at present, most people don't pay attention to that problem. Besides, even if they want to check the degree of dirtiness inside the noise reduction box, it is difficult to do so, because most of the noise reduction boxes used presently are enclosed ones and can't be disassembled and reassembled or are difficult to disassemble and reassemble, and it is highly likely that the performance of the device containing the noise reduction box is compromised after the noise reduction box is reassembled, since the noise reduction box involves many parts.

A medical respirator is described in CN101850146. The medical respirator comprises a housing, a ventilation unit and an airway that is located inside the housing. The housing is perforated with an air inlet and an air outlet. A replaceable filter device is arranged at the air inlet. The filter device filters a part of the suspended particles contained in the air that flows into the respirator. The respirator can comprise a particle detector arranged at the outside of the housing. The particle detector is used to detect the content of suspended particles in the air outside the respirator.

CN104180444 shows a combined domestic air cleaner that cleans indoor air in a room and simultaneously provides the room with fresh outdoor air. The combined air cleaner comprises a housing, an air supply port and at least one filter module. The combined air cleaner is connected to a fresh air hole through a fresh air pipe and the fresh air hole is connected to outdoor fresh air. A fan is arranged in the housing of the combined air cleaner. The fan is arranged in a noise reduction device. A first filter module is arranged in front of the fan and a second filter module is arranged after the fan.

### SUMMARY

In view of the above problems, the object of the present disclosure is to provide a noise reduction box having an internal dirtiness detection function and a ventilation therapeutic device including the noise reduction box.

In order to attain the above object, in an aspect, the present disclosure provides a noise reduction box, which comprises a shell and a detection assembly, wherein the inside of the shell defines a cavity, the shell is provided with an air inlet and an air outlet that are in communication with the cavity; and the detection assembly is mounted on the shell and wherein the detection assembly is adapted to detect the degree to which the inside of the cavity is dirty, so that the noise reduction box can be cleaned and serviced timely, wherein the detection assembly comprises a dirt adsorption component and the dirt adsorption component is adapted to absorb the pollutants in the air in the cavity and to develop the color to different degrees according to the amount of the absorbed pollutants.

Optionally, the dirt adsorption component is a transparent wall, which is formed as a part of the shell.

Optionally, the detection assembly comprises a light source, which is arranged in the cavity to illuminate the transparent wall.

Optionally, the cavity is provided with a partition assembly therein, which divides the cavity into an air intake cavity, a fan mounting cavity and an air discharge cavity, wherein the air inlet is in communication with the air intake cavity, the air outlet is in communication with the air discharge cavity, the air intake cavity is in communication with the fan mounting cavity through a first through hole formed in the partition assembly, the fan mounting cavity is in communication with the air discharge cavity through a second through hole formed in the partition assembly, the light source is arranged in the air intake cavity, and the transparent wall is formed as a part of the shell for defining the air intake cavity.

Optionally, the dirt adsorption component is air-permeable cotton, which is at least partially arranged in the cavity and can be taken out from the cavity.

Optionally, the detection assembly comprises a mounting component for mounting the air-permeable cotton, the shell is provided with a mounting port in communication with the cavity, and the mounting component is removably mounted in the mounting port so that the air-permeable cotton is positioned in the cavity.

Optionally, the mounting component is a plate-shaped component partially inserted into the cavity through the mounting port, an annular groove is arranged on the mounting component around the periphery of the mounting component and divides the mounting component into an insertion portion and a mounting portion in an insertion direction, the air-permeable cotton is mounted on the mounting portion, and the detection assembly comprises a seal ring embedded in the annular groove and configured to be in seal-fit with the periphery of the mounting port when the mounting portion is inserted into the cavity.

Optionally, the cavity is provided with a partition assembly therein, which divides the cavity into an air intake cavity, a fan mounting cavity and an air discharge cavity, wherein the air inlet is in communication with the air intake cavity, the air outlet is in communication with the air discharge cavity, the air intake cavity is in communication with the fan mounting cavity through a first through hole formed in the partition assembly, the fan mounting cavity is in communication with the air discharge cavity through a second through hole formed in the partition assembly, and the mounting port is configured to be in communication with the air intake cavity.

Optionally, the air-permeable cotton is plate-shaped, wherein
the mounting portion is arranged to be inserted into the cavity through the mounting port in a direction perpendicular to an air flow direction in the cavity, the mounting portion is formed as an annular mounting frame, and the air-permeable cotton is embedded in the mounting frame; or
the mounting portion is arranged to be inserted into the cavity through the mounting port in a direction parallel to the air flow direction in the cavity, and a top surface of the mounting portion is provided with a mounting groove for embedding the air-permeable cotton therein.

Optionally, the cavity is provided with a guide structure for guiding the insertion of the mounting portion and supporting the mounting portion therein.

Optionally, the shell comprises an upper shell and a lower shell, the noise reduction box comprises a sealing element sandwiched between the upper shell and the lower shell, and the detection assembly is mounted on the upper shell or the lower shell.

In another aspect, the present disclosure provides a ventilation therapeutic device, which comprises the noise reduction box described above.

A special detection assembly for detecting the degree to which the inside of the noise reduction box is dirty is mounted on the noise reduction box in the present disclosure, so that the degree to which the inside of the noise reduction box is dirty can be clearly observed by means of the detection assembly, without it being necessary to disassemble the noise reduction box, and same is simple to operate and convenient for checking.

Other features and advantages of the present disclosure will be further detailed in the following embodiments.

### BRIEF DESCRIPTION OF THE DRAWINGS

The accompanying drawings are provided herein to facilitate further understanding on the present disclosure and constitute a part of this document. They are used in conjunction with the following embodiments to explain the present disclosure, but are not intended to constitute any limitation to the present disclosure. In the figures:
Fig. 1 is a perspective view of an embodiment of the noise reduction box with a detection assembly mounted on the shell according to the present disclosure;
Fig. 2 is a schematic view of extracting the detection assembly in Fig. 1 from the shell;
Fig. 3 is an exploded view of the structure in Fig. 2;
Fig. 4 is a schematic view of the upper shell in Fig. 3 from another view angle;
Fig. 5 is a schematic view of the lower shell in Fig. 3 from another view angle;
Fig. 6 is a schematic structural view of the mounting component in Fig. 3;
Fig. 7 is a front view of the structure in Fig. 1;
Fig. 8 is a sectional view A-A of the structure in Fig. 7;
Fig. 9 is a perspective view of the structure in Fig. 8;
Fig. 10 is another perspective view of an embodiment of the noise reduction box with a detection assembly mounted on the shell according to the present disclosure;
Fig. 11 is a schematic view of extracting the detection assembly in Fig. 10 from the shell;
Fig. 12 is an exploded view of the structure in Fig. 11;
Fig. 13 is a schematic view of the upper shell in Fig. 12 from another view angle;
Fig. 14 is a side view of the structure shown in Fig. 10;
Fig. 15 is a sectional view B-B of the structure in Fig. 14;
Fig. 16 is a perspective view of the structure in Fig. 15;
Fig. 17 is a perspective view of yet another embodiment of the noise reduction box according to the present disclosure;
Fig. 18 is a side view of the structure shown in Fig. 17;
Fig. 19 is an exploded view of the structure in Fig. 17;
Fig. 20 is a schematic view of the upper shell in Fig. 19 from another view angle;
Fig. 21 is a sectional view C-C of the structure shown in Fig. 18;
Fig. 22 is a perspective view of the structure in Fig. 21.

### REFERENCE NUMBERS

10 - shell; 101 - air inlet; 102 - air outlet; 103 - mounting port; 11 - air intake cavity; 111 - guide structure; 12 - fan mounting cavity; 13 - air discharge cavity; 14 - first partition; 141 - first through hole; 142 - mounting base; 15 - second partition; 151 - second through hole; 16 - third partition; 17 - fourth partition; 18 - upper shell; 19 - lower shell; 20 - transparent wall; 21 - light source; 30 - air-permeable cotton; 31 - mounting component; 311 - annular groove; 312 - insertion portion; 313 - mounting portion; 314 - annular boss; 315 - mounting groove; 32 - seal ring; 40 - sealing element.

### DETAILED DESCRIPTION

Hereunder some embodiments of the present disclosure will be detailed with reference to the accompanying drawings. It may be understood that the embodiments described herein are only provided to describe and explain the present disclosure, but are not intended to constitute any limitation to the present disclosure.

In the present disclosure, unless otherwise specified, the terms that denote the orientations are used as follows, for example: "top" and "bottom" usually refer to orientations in the state of mounting and use; "inside" and "outside" refer to inside and outside in relation to the profiles of the components.

In an aspect, the present disclosure provides a noise reduction box, which comprises a shell 10 and a detection assembly, wherein the inside of the shell 10 defines a cavity, the shell 10 is provided with an air inlet 101 and an air outlet 102 that are in communication with the cavity; and the detection assembly is mounted on the shell 10 to detect the degree to which the inside of the cavity is dirty.

A detection assembly is mounted, specifically for detecting the degree to which the inside of the noise reduction box is dirty, on the noise reduction box in the present disclosure, so that the degree to which the inside of the noise reduction box is dirty can be clearly observed by means of the detection assembly, without it being necessary to disassemble the noise reduction box, and same is simple to operate and convenient for checking.

The above-mentioned detection assembly may be implemented in various ways, as long as it can detect the degree to which the inside of the cavity is dirty and is convenient for the user to observe or learn about the degree to which the inside of the cavity is dirty.

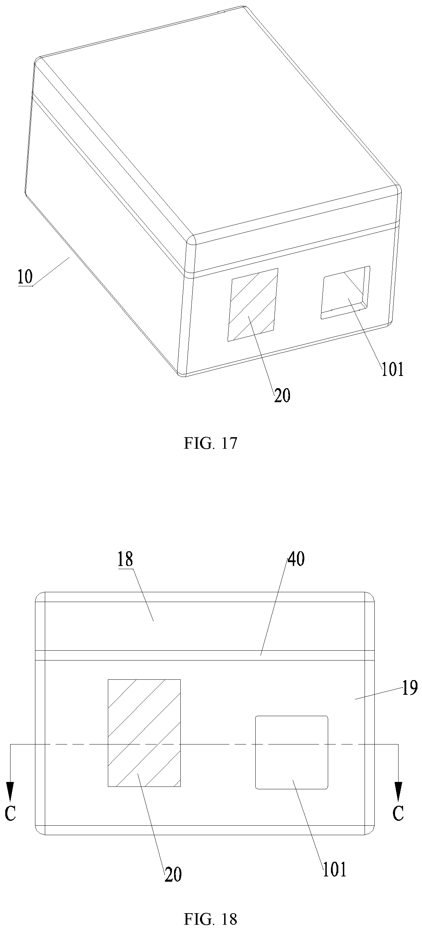

According to an embodiment of the detection assembly in the present disclosure, the detection assembly comprises a dirt adsorption component. The above-mentioned dirt adsorption component may be understood as a component that can absorb the pollutants in the air in the cavity and develop the color to different degrees according to the amount of the absorbed pollutants. Specifically, as shown in Figs. 17-22, the dirt adsorption component may be a transparent wall 20, which is formed as a part of the shell 10. By providing the transparent wall 20, dirt will adhere to and accumulate on the transparent wall 20 as the noise reduction box is used, and the transparency of the transparent wall 20 will change accordingly. The higher the degree of the dirtiness is inside the cavity, the lower the transparency of the transparent wall 20 is. Therefore, the degree of dirtiness inside the noise reduction box may be judged according to the level of transparency of the transparent wall 20, thus the degree of the dirtiness inside the noise reduction box can be identified easily without disassembling the noise reduction box, and it is convenient for the user to decide whether to clean and service the noise reduction box. Furthermore, the detection assembly may further comprise a light source 21, which may be arranged in the cavity to illuminate the transparent wall 20. By providing the light source 21, the level of transparency of the transparent wall 20 can be clearly observed in all cases, so that the degree of dirtiness inside the cavity can be ascertained more accurately.

In addition, in view that a fan is usually accommodated in the cavity, preferably the cavity is divided into several regions to facilitate the mounting of the fan and the detection of the degree of dirtiness therein. Specifically, a partition assembly may be arranged inside the cavity to divide the cavity into an air intake cavity 11, a fan mounting cavity 12 and an air discharge cavity 13, wherein the air inlet 101 is in communication with the air intake cavity 11, the air outlet 102 is in communication with the air discharge cavity 13, the air intake cavity 11 is in communication with the fan mounting cavity 12 through a first through hole 141 formed in the partition assembly, and the fan mounting cavity 12 is in communication with the air discharge cavity 13 through a second through hole 151 formed in the partition assembly.

During use, external gas (e.g., air) enters into the air intake cavity 11 through the air inlet 101, and then enters into the fan mounting cavity 12 through the first through hole 141. The gas entering into the fan mounting cavity 12 enters into the fan, then enters into the air discharge cavity 13 through the outlet of the fan and the second through hole 151, and finally is discharged through the air outlet 102.

From the above description, it can be seen that the air intake cavity 11 may be the cavity that can best represent the degree of dirtiness in the cavity. Therefore, in order to ensure the accuracy of the detection of the degree of dirtiness, a light source 21 may be arranged in the air intake cavity 11, and the transparent wall 20 is formed as a part of the shell 10 for defining the air intake cavity 11. It can be noted that the air intake cavity 11 may be defined by the transparent wall 20 in entirety or in part. In addition, the transparent wall 20 may be arranged on the shell 10 in any way, but it is advantageous to ensure that there is no clearance between the transparent wall 20 and the shell 10, so as to avoid the loss of the gas in the cavity through the clearance.

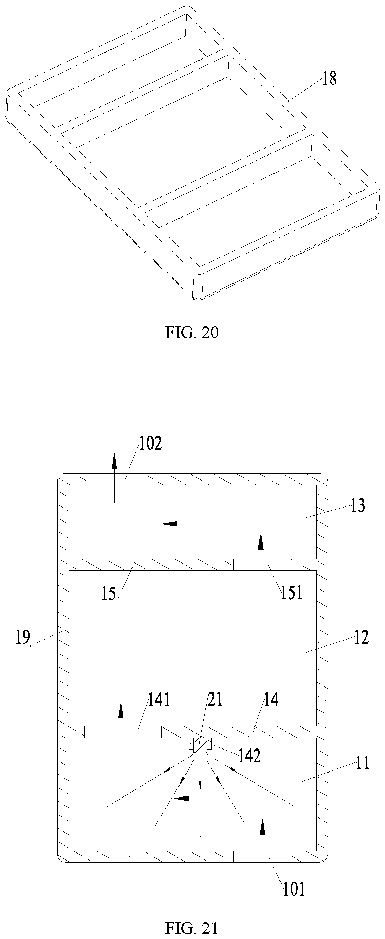

The above-mentioned air intake cavity 11, fan mounting cavity 12 and air discharge cavity 13 may be arranged appropriately in any way. According to an embodiment of the present disclosure, as shown in Fig. 21, the air inlet 101 and the air outlet 102 are respectively located in the opposite side walls of the shell 10, and the partition assembly divides the cavity into an air intake cavity 11, a fan mounting cavity 12 and an air discharge cavity 13 sequentially in a direction from the air inlet 101 to the air outlet 102.

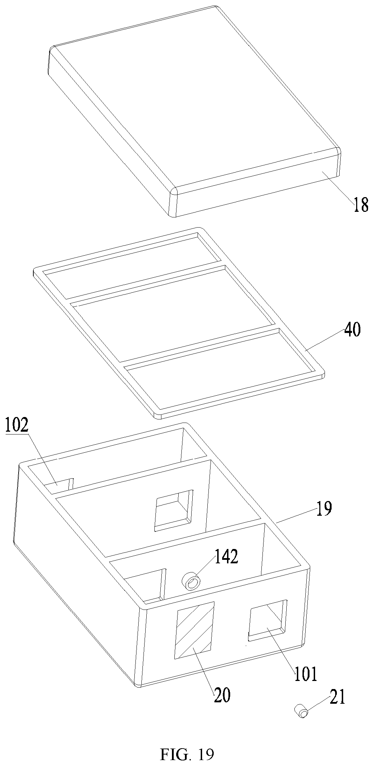

The above-mentioned partition assembly may have any suitable composition and structure. According to an embodiment of the partition assembly in the present disclosure, as shown in Figs. 19-22, the partition assembly may comprise a first partition 14 and a second partition 15 that are spaced apart from each other, wherein the first partition 14 is located between the air intake cavity 11 and the fan mounting cavity 12; the first through hole 141 is arranged in the first partition 14; the second partition 15 is located between the fan mounting cavity 12 and the air discharge cavity 13; and the second through hole 151 is arranged in the second partition 15. It can be understood that the air intake cavity 11 and the fan mounting cavity 12 are separated by the first partition 14, and the fan mounting cavity 12 and the air discharge cavity 13 are separated by the second partition 15. The shape and arrangement of the first partition 14 and the second partition 15 may be designed according to the actual requirements of the cavities (i.e., the air intake cavity 11, the fan mounting cavity 12 and the air discharge cavity 13). Of course, the design of the internal space of the cavity is not limited to the above-mentioned design; instead, the internal space of the cavity may be designed differently according to the actual requirements.

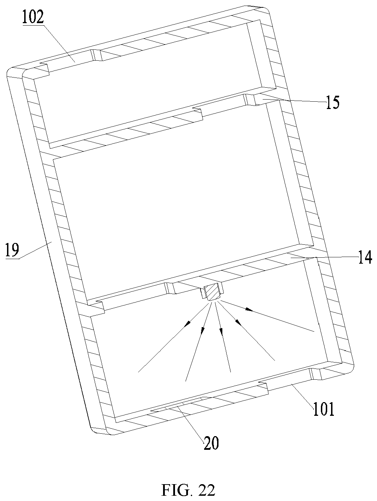

In the above embodiment, in order to improve the convenience and clarity of observation of the transparent wall 20, as shown in Figs. 21 and 22, the transparent wall 20 may be located on a side wall of the shell 10 opposite to the first partition 14, and the light source 21 may be mounted on the first partition 14.

The light source 21 may be mounted on the first partition 14 in any appropriate way. For example, a mounting base 142 for mounting the light source 21 may be provided on the first partition 14. Specifically, as shown in Figs. 19 and 21, the light source 21 may be a columnar lamp bead, and the mounting base 142 may be an annular structure extending from the first partition 14 toward the side where the transparent wall 20 is located, and the light source 21 is embedded in the annular structure. Of course, the light source 21 may be further connected to the mounting base 142 by bonding, etc., so as to improve the firmness and reliability of the mounting of the light source 21.

In the present disclosure, the dirt adsorption component may be air-permeable cotton 30, which is at least partially arranged in the cavity and can be taken out from the cavity. In order to facilitate color development, the air-permeable cotton 30 is preferably in a light color, such as white, cream, light pink, etc. **In** addition, in order to accurately reflect the degree of dirtiness of the light-colored air-permeable cotton, a gradient color bar chart may be worked out according to the degree of dirtiness. During use, the mounting component 31 may be taken out from the mounting port 103 at any time to observe the color of the light-colored air-permeable cotton 30 and compare it with the gradient color bar chart to identify the degree of dirtiness, thereby the degree of dirtiness inside the noise reduction box can be judged, so that the user can decide conveniently whether to clean and service the noise reduction box.

Furthermore, in order to facilitate the mounting of the air-permeable cotton 30, the detection assembly may further comprise a mounting component 31 for mounting the air-permeable cotton 30. That is to say, the air-permeable cotton 30 may be mounted on the mounting component 31 and then mounted on the shell 10 via the mounting component 31. **In** order to enable the air-permeable cotton 30 to be positioned in the cavity and taken out from the cavity, the shell 10 may be provided with a mounting port 103 in communication with the cavity, and the mounting component 31 may be removably mounted in the mounting port 103. When the mounting component 31 is mounted in the mounting port 103, the air-permeable cotton 30 may be positioned in the cavity to detect the degree of dirtiness. To check the degree of dirtiness inside the cavity, the mounting component 31 may be removed from the mounting port 103 and the developed color of the air-permeable cotton 30 may be observed.

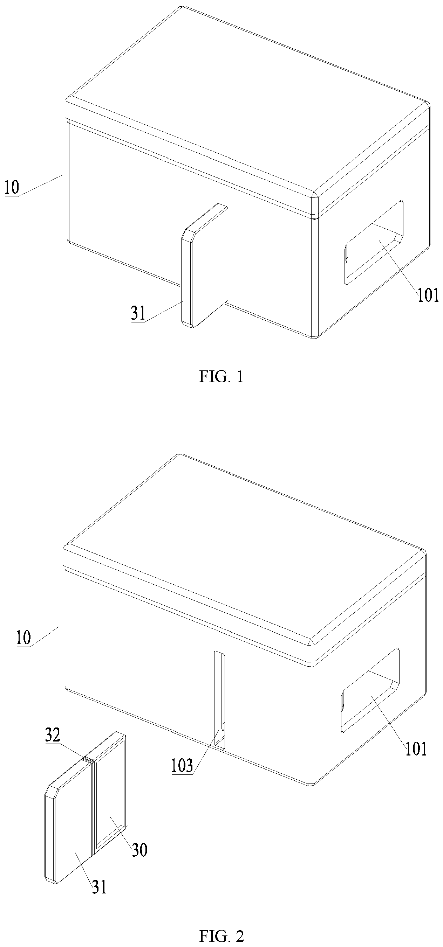

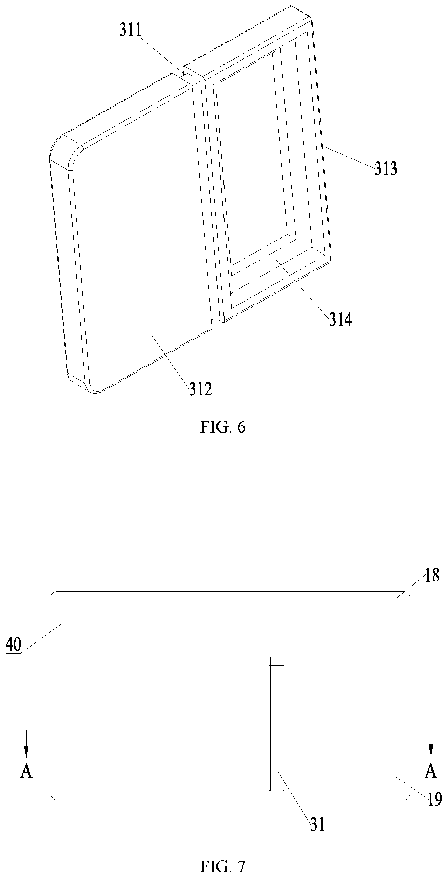

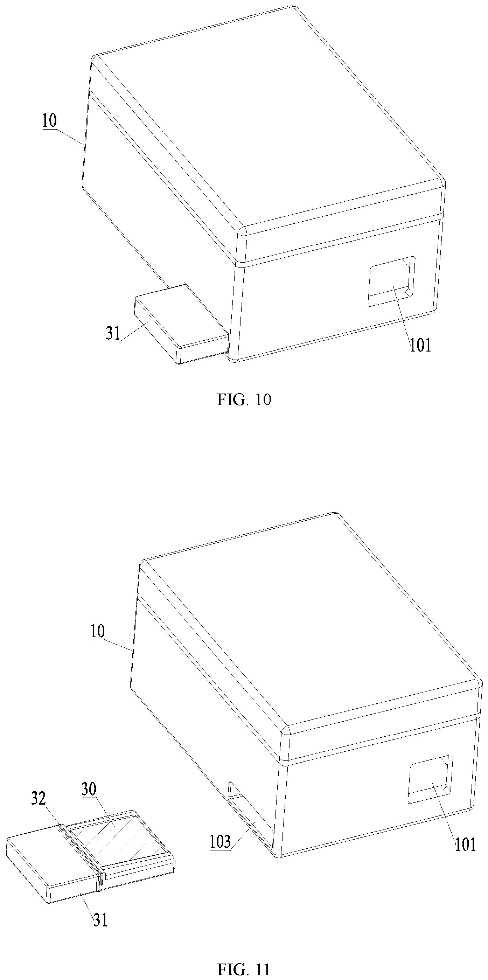

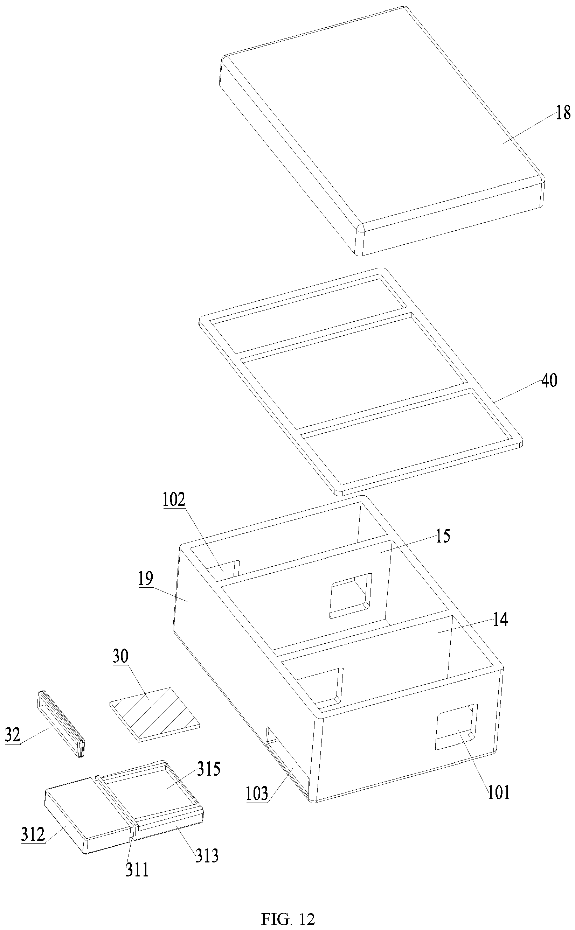

As for the specific structure of the mounting component 31 and the mounting method of the air-permeable cotton 30, according to an embodiment of the present disclosure, the mounting component 31 may be a plate-shaped component partially inserted into the cavity through the mounting port 103 (see Figs. 1 and 10), and an annular groove 311 is arranged on the mounting component 31 around the periphery of the mounting component 31 and divides the mounting component 31 into an insertion portion 312 and a mounting portion 313 in an insertion direction (i.e., the direction in which the mounting port 103 is arranged) (see Figs. 6 and 12), the air-permeable cotton 30 is mounted on the mounting portion 313 (see Figs. 2 and 11), and the detection assembly may further comprise a seal ring 32 embedded in the annular groove 311 and configured to be in seal-fit with the periphery of the mounting port 103 when the mounting portion 313 is inserted into the cavity (see Fig. 2, 9, 11 and 16).

To enhance the reliability of the coupling between the seal ring 32 and a sealing element 31, the seal ring 32 may be connected to the annular groove 311 by secondary encapsulation or bonding.

It can be understood that the mounting component 31 and the air-permeable cotton 30 mounted thereon may be inserted into the mounting port 103 and taken out from the mounting port 103 as an integral piece, wherein the insertion portion 312 of the mounting component 31 provides a force applying part for the user to insert the mounting component 31 into the mounting port 103 or extract the mounting component 31 from the mounting port 103 by holding the insertion portion 312 of the mounting component 31; when the mounting component 31 is inserted into the mounting port 103, the mounting portion 313 with the air-permeable cotton 30 enters into the cavity, while the insertion portion 312 is located outside the mounting port 103, and the seal ring 32 between the mounting portion 313 and the insertion portion 312 is in seal-fit with the periphery of the mounting port 103, so as to prevent the loss of the air in the cavity.

Specifically, the mounting component 31 may be formed as a square plate-shaped component, and the annular groove 311 divides the mounting component 31 into an insertion portion 312 and a mounting portion 313 in a lengthwise direction. The air-permeable cotton 30 may be plate-shaped.

Please see the embodiment shown in Figs. 1-9. The mounting portion 313 may be arranged to be inserted into the cavity through the mounting port 103 in a direction perpendicular to the direction of the air flow through the cavity (i.e., the vertical direction shown in Fig. 1, wherein the air flow direction is horizontal, i.e., a direction from the air inlet 101 to the air outlet 102), and the mounting portion 313 is formed as an annular mounting frame, the air-permeable cotton 30 is embedded in the annular mounting frame, and the gas in the cavity can flow through the air-permeable cotton 30. As shown in Figs. 6 and 2, an annular boss 314 may be provided on an inner peripheral surface of the mounting frame, and the edges of the air-permeable cotton 30 may be bonded to the annular boss 314. Alternatively, the air-permeable cotton 30 may be formed integrally with the mounting portion 313 by insert molding.

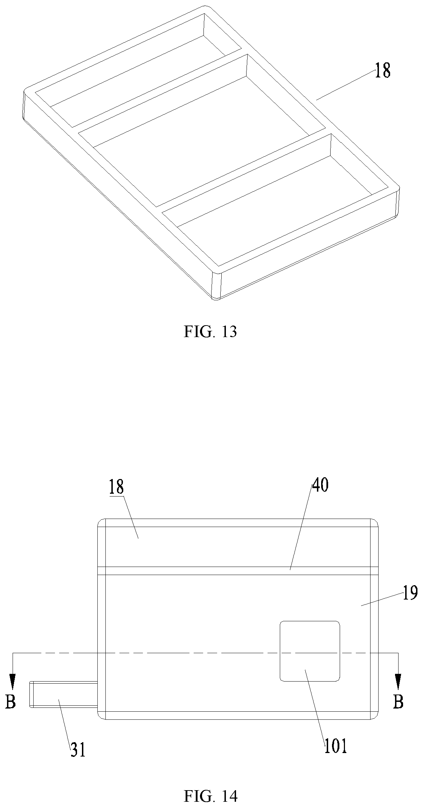

Please see the embodiment shown in Figs. 10-16. The mounting portion 313 may be arranged to be inserted into the cavity through the mounting port 103 in a direction parallel to the direction of the air flow through the cavity (i.e., the horizontal direction shown in Fig. 14), and the top surface of the mounting portion 313 is provided with a mounting groove 315 for embedding the air-permeable cotton 30. As shown in Figs. 11 and 12, in order to enhance the reliability of the mounting of the air-permeable cotton 30, the air-permeable cotton 30 may be bonded to a bottom surface of the mounting groove 315.

It can be noted that the air-permeable cotton 30 and the mounting portion 313 may be mounted and connected in different ways, not limited to the way described above. The air-permeable cotton 30 may be detachably connected with the mounting portion 313, for example, by means of a snap-in structure, so that the air-permeable cotton 30 may be replaced conveniently.

In addition, to facilitate the insertion and extraction of the mounting component 31 while avoiding any sway of the mounting component 31 after it is inserted into the mounting port 103, a guide structure 111 for guiding the insertion of the mounting portion 313 and supporting the mounting portion 313 may be arranged in the cavity.

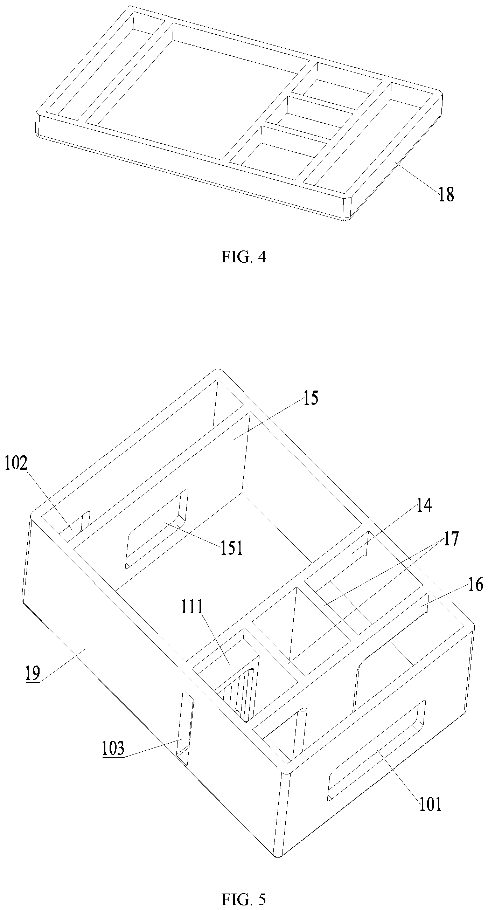

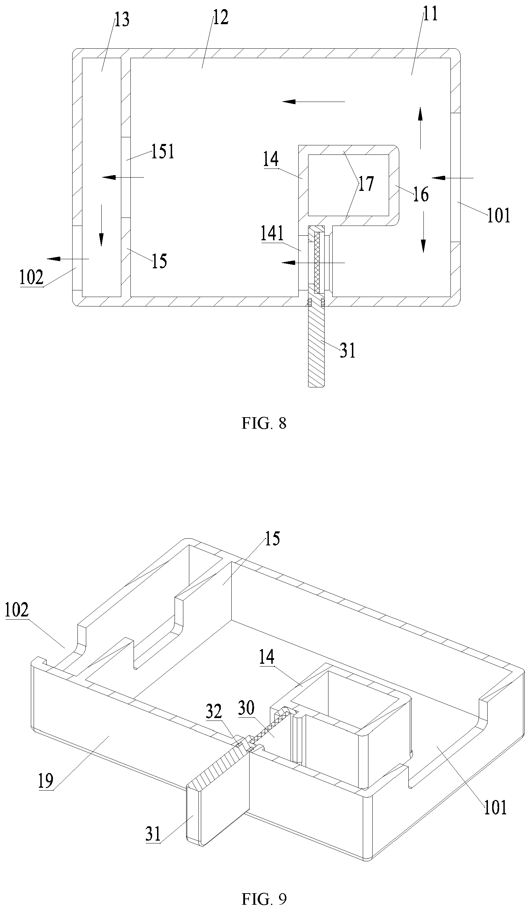

For the embodiment shown in Figs. 1-9, specifically, as shown in Figs. 5 and 9, the guide structure 111 may be formed as a frame, and the inner peripheral surface of the frame may be provided with guide grooves that match the mounting portion 313 of the mounting component 31. After the mounting portion 313 enters into the mounting port 103, a top edge and a bottom edge of the mounting portion 313 enter into top guide groove and bottom guide groove of the frame and move further along the top guide groove and bottom guide groove. Finally, a right edge of the mounting portion 313 enters into a right guide groove of the frame and stops the movement; at that point, the seal ring 32 on the mounting portion 31 is right in seal-fit with the periphery of the mounting port 103, and the mounting portion 313 entering into the cavity will not sway under a limiting effect of the guide grooves.

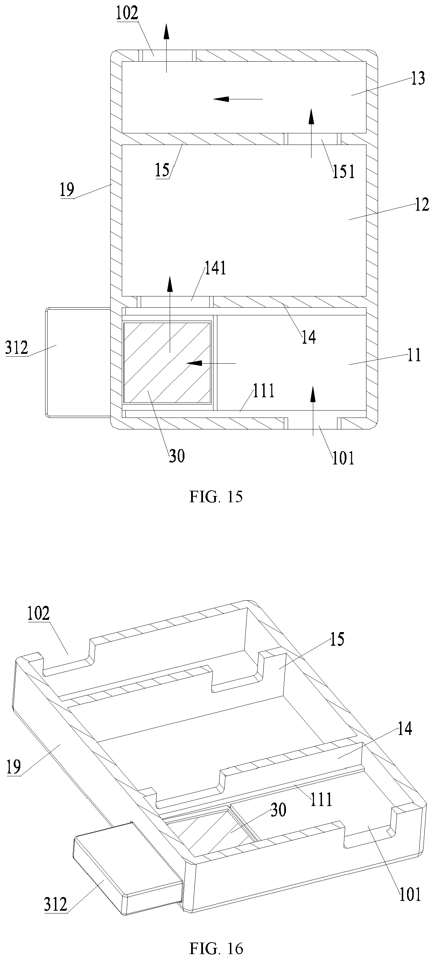

For the embodiment shown in Figs. 10-16, specifically, as shown in Figs. 15 and 16, the guide structure 111 may be formed as two guide rails that are parallel to each other, spaced apart from each other, and extend in the insertion direction of the mounting component 31. After entering into the mounting port 103, the mounting portion 313 of the mounting component 31 can move along the guide rails, and the spacing between the guide rails and the bottom wall of the shell 10 may match the thickness of the mounting portion 313, so that the mounting portion 313 can be positioned between the guide rails and the shell 10 and thereby a limiting effect is achieved; in addition, a limiting structure for limiting the further movement of the mounting portion 313 may be arranged on the guide rails or on the bottom wall of the shell 10, so as to limit the insertion position of the mounting portion 313 and ensure the seal-fit between the seal ring 32 and the mounting port 103.

It can be noted that the structures of the air-permeable cotton 30 and the mounting portion 31, the mounting method for the air-permeable cotton 30 and the mounting portion 31, and the mounting method for the mounting portion 31 and the shell 10 in the present disclosure are not limited to those in the embodiments described above. Any structure and mounting method may be used as long as they can achieve the desired function. However, any such structure and mounting method shall be deemed as falling in the scope of protection of the present disclosure.

In addition, in the case that the cavity is divided by the partition assembly into an air intake cavity 11, a fan mounting cavity 12 and an air discharge cavity 13, the mounting port 103 may be configured to be in communication with the air intake cavity 11.

In the present disclosure, it can be noted that the cavity may be divided by the partition assembly in any appropriate way. Of course, the inside of the cavity may be designed in other ways to realize the detection of the degree of dirtiness in the cavity and the communication between the cavities more reasonably and advantageously.

Specifically, for example, in the embodiment shown in Fig. 5, the partition assembly comprises a first partition 14, a second partition 15, a third partition 16 and fourth partitions 17, wherein the first partition 14 and the second partition 15 are spaced apart from each other and divide the cavity into an air intake cavity 11, a fan mounting cavity 12 and an air discharge cavity 13 sequentially in the air flow direction; the first partition 14 is provided with a first through hole 141 for communication between the air intake cavity 11 and the fan mounting cavity 12; the second partition 15 is provided with a second through hole 151 for communication between the fan mounting cavity 12 and the air discharge cavity 13; the third partition 16 and the fourth partitions 17 are arranged in the air intake cavity 11; the third partition 16 and the first partition 14 are spaced apart from each other; two fourth partitions 17 are connected in a spaced apart manner between the third partition 16 and the first partition 14 so as to divide the air intake cavity 11 into two small cavities that are spaced apart from each other in a left-right direction; and the first partition 14 is provided with two first through holes 141 that are in communication with the two small cavities respectively. In such a case, the mounting portion 313 of the mounting component 31 may be located at one of the first through holes 141. During use, as shown in Fig. 8, the gas entering into the air intake cavity 11 through the air inlet 101 may flow into the upper small cavity and the lower small cavity respectively, the gas flowing into the upper small cavity may directly enter into the fan mounting cavity 12 through the upper first through hole 141, while the gas flowing into the lower small cavity may pass through the air-permeable cotton 30 and enter into the fan mounting cavity 12 through the lower first through hole 141. By branching the gas entering into the air intake cavity 11, a part of the gas may be introduced specially for the detection of the degree of dirtiness on a premise of ensuring gas circulation, so that the detection of the degree of dirtiness is more accurate and reliable.

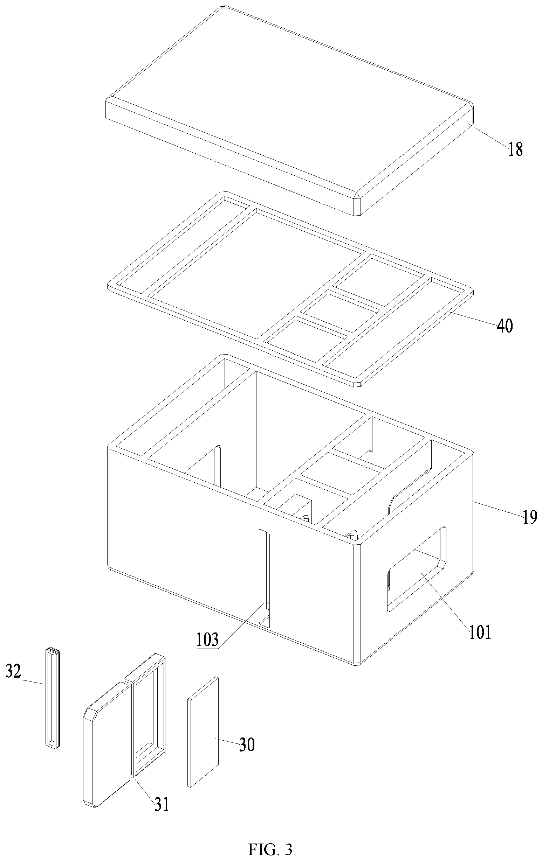

**In** the present disclosure, the shell 10 may have any appropriate structure, and there is no restriction on the specific structure of the shell 10 in the present disclosure. For example, as shown in Fig. 3, the shell 10 may comprise an upper shell 18 and a lower shell 19, which may be fixedly connected, detachably connected or rotatably connected with each other. **In** addition, the noise reduction box may further comprise a sealing element 40 sandwiched between the upper shell 18 and the lower shell 19 to ensure the sealing between the upper shell 18 and the lower shell 19. Depending the specific structure of the upper shell 18 and the low shell 19, the detection assembly may be mounted on the upper shell 18 or the low shell 19. **In** an embodiment of the shell 10 in the present disclosure, the cavity is mainly defined by the lower shell 19; thus, the detection assembly may be mounted on the lower shell 19.

**In** another aspect, the present disclosure provides a ventilation therapeutic device, which comprises the noise reduction box described above.

The ventilation therapeutic device may be a ventilator, and a fan may be arranged in the noise reduction box.

While some preferred embodiments of the present disclosure are described above with reference to the accompanying drawings, the present disclosure is not limited to the details in those embodiments. Those skilled in the art can make various simple modifications and variations to the technical scheme of the present disclosure, without departing from the technical concept of the present disclosure. However, all these simple modifications and variations shall be deemed as falling in the scope of protection of the present disclosure.

**In** addition, it can be noted that the specific technical features described in the above embodiments may be combined in any appropriate form, provided that there is no conflict among them. To avoid unnecessary repetition, the possible combinations are not described specifically in the present disclosure.

Moreover, different embodiments of the present disclosure may also be combined freely as required, as long as the combinations don't deviate from the ideal of the present disclosure. However, such combinations shall also be deemed as being disclosed in the present disclosure.

## Claims

1. A noise reduction box, comprising a shell (10) and a detection assembly, wherein the inside of the shell (10) defines a cavity, the shell (10) is provided with an air inlet (101) and an air outlet (102) that are in communication with the cavity, and the detection assembly is mounted on the shell (10); **characterized in that**
the detection assembly is adapted to detect the degree to which the inside of the cavity is dirty, so that the noise reduction box can be cleaned and serviced timely, wherein the detection assembly comprises a dirt adsorption component and the dirt adsorption component is adapted to absorb the pollutants in the air in the cavity and to develop the color to different degrees according to the amount of the absorbed pollutants.

2. The noise reduction box of claim 1, wherein the dirt adsorption component is a transparent wall (20), which is formed as a part of the shell (10).

3. The noise reduction box of claim 2, wherein the detection assembly comprises a light source (21), which is arranged in the cavity to illuminate the transparent wall (20).

4. The noise reduction box of claim 2, wherein the cavity is provided with a partition assembly therein, which divides the cavity into an air intake cavity (11), a fan mounting cavity (12) and an air discharge cavity (13), wherein the air inlet (101) is in communication with the air intake cavity (11), the air outlet (102) is in communication with the air discharge cavity (13), the air intake cavity (11) is in communication with the fan mounting cavity (12) through a first through hole (141) formed in the partition assembly, the fan mounting cavity (12) is in communication with the air discharge cavity (13) through a second through hole (151) formed in the partition assembly, the light source (21) is arranged in the air intake cavity (11), and the transparent wall (20) is formed as a part of the shell (10) for defining the air intake cavity (11).

5. The noise reduction box of claim 1, wherein the dirt adsorption component is air-permeable cotton (30), which is at least partially arranged in the cavity and can be taken out from the cavity.

6. The noise reduction box of claim 5, wherein the detection assembly comprises a mounting component (31) for mounting the air-permeable cotton (30), the shell (10) is provided with a mounting port (103) in communication with the cavity, and the mounting component (31) is removably mounted in the mounting port (103) so that the air-permeable cotton (30) is positioned in the cavity.

7. The noise reduction box of claim 6, wherein
the mounting component (31) is a plate-shaped component partially inserted into the cavity through the mounting port (103), an annular groove (311) is arranged on the mounting component (31) around the periphery of the mounting component (31) and divides the mounting component (31) into an insertion portion (312) and a mounting portion (313) in an insertion direction, the air-permeable cotton (30) is mounted on the mounting portion (313), and the detection assembly comprises a seal ring (32) embedded in the annular groove (311) and configured to be in seal-fit with the periphery of the mounting port (103) when the mounting portion (313) is inserted into the cavity; and/or
the cavity is provided with a partition assembly therein, which divides the cavity into an air intake cavity (11), a fan mounting cavity (12) and an air discharge cavity (13), wherein the air inlet (101) is in communication with the air intake cavity (11), the air outlet (102) is in communication with the air discharge cavity (13), the air intake cavity (11) is in communication with the fan mounting cavity (12) through a first through hole (141) formed in the partition assembly, the fan mounting cavity (12) is in communication with the air discharge cavity (13) through a second through hole (151) formed in the partition assembly, and the mounting port (103) is configured to be in communication with the air intake cavity (11).

8. The noise reduction box of claim 7, wherein the air-permeable cotton (30) is plate-shaped, wherein
the mounting portion (313) is arranged to be inserted into the cavity through the mounting port (103) in a direction perpendicular to an air flow direction in the cavity, the mounting portion (313) is formed as an annular mounting frame, and the air-permeable cotton (30) is embedded in the mounting frame; or
the mounting portion (313) is arranged to be inserted into the cavity through the mounting port (103) in a direction parallel to the air flow direction in the cavity, and a top surface of the mounting portion (313) is provided with a mounting groove (315) for embedding the air-permeable cotton (30) therein.

9. The noise reduction box of claim 7, wherein the cavity is provided with a guide structure (111) for guiding the insertion of the mounting portion (313) and supporting the mounting portion (313) therein.

10. The noise reduction box of any of claims 1-9, wherein the shell (10) comprises an upper shell (18) and a lower shell (19), the noise reduction box comprises a sealing element (40) sandwiched between the upper shell (18) and the lower shell (19), and the detection assembly is mounted on the upper shell (18) or the lower shell (19).

11. A ventilation therapeutic device, comprising the noise reduction box of any of claims 1-10.

## Patentansprüche

1. Schallschluckbox, umfassend ein Gehäuse (10) und eine Erfassungsbaugruppe, wobei das Innere des Gehäuses (10) einen Hohlraum definiert, das Gehäuse (10) mit einem Lufteinlass (101) und einem Luftauslass (102) versehen ist, die mit dem Hohlraum in Verbindung stehen, und die Erfassungsbaugruppe an dem Gehäuse (10) angebracht ist; **dadurch gekennzeichnet, dass**
die Erfassungsbaugruppe ausgebildet ist, um den Verschmutzungsgrad des Inneren des Hohlraums zu erfassen, so dass die Schallschluckbox zeitgerecht gereinigt und gewartet werden kann, wobei die Erfassungsbaugruppe eine Schmutzadsorptionskomponente umfasst und die Schmutzadsorptionskomponente ausgebildet ist, um die Schmutzstoffe in der Luft im Hohlraum zu absorbieren und je nach Menge der absorbierten Schadstoffe in unterschiedlichem Maße eine Farbe zu entwickeln.

2. Schallschluckbox nach Anspruch 1, wobei die Schmutzadsorptionskomponente eine transparente Wand (20) ist, die als Teil des Gehäuses (10) ausgebildet ist.

3. Schallschluckbox nach Anspruch 2, wobei die Erfassungsbaugruppe eine Lichtquelle (21) umfasst, die in dem Hohlraum angeordnet ist, um die transparente Wand (20) zu beleuchten.

4. Schallschluckbox nach Anspruch 2, wobei der Hohlraum mit einer darin befindlichen Trennwandanordnung versehen ist, die den Hohlraum in einen Lufteinlasshohlraum (11), einen Lüfterbefestigungshohlraum (12) und einen Luftauslasshohlraum (13) unterteilt, wobei der Lufteinlass (101) mit dem Lufteinlasshohlraum (11) in Verbindung steht, der Luftauslass (102) mit dem Luftauslasshohlraum (13) in Verbindung steht, der Lufteinlasshohlraum (11) mit dem Lüfterbefestigungshohlraum (12) über ein erstes, in der Trennwandanordnung ausgebildetes Durchgangsloch (141) in Verbindung steht, der Lüfterbefestigungshohlraum (12) mit dem Luftauslasshohlraum (13) über ein zweites, in der Trennwandanordnung ausgebildetes Durchgangsloch (151) in Verbindung steht, die Lichtquelle (21) in dem Lufteinlasshohlraum (11) angeordnet ist, und die transparente Wand (20) als Teil des Gehäuses (10) zum Definieren des Lufteinlasshohlraums (11) ausgebildet ist.

5. Schallschluckbox nach Anspruch 1, wobei die Schmutzadsorptionskomponente luftdurchlässige Baumwolle (30) ist, die zumindest teilweise in dem Hohlraum angeordnet ist und aus dem Hohlraum herausgenommen werden kann.

6. Schallschluckbox nach Anspruch 5, wobei die Erfassungsbaugruppe eine Befestigungskomponente (31) zum Befestigen der luftdurchlässigen Baumwolle (30) umfasst, das Gehäuse (10) mit einer Befestigungsöffnung (103) versehen ist, die mit dem Hohlraum in Verbindung steht, und die Befestigungskomponente (31) entfernbar in der Befestigungsöffnung (103) befestigt ist, so dass die luftdurchlässige Baumwolle (30) in dem Hohlraum positioniert ist.

7. Schallschluckbox nach Anspruch 6, wobei
die Befestigungskomponente (31) ein plattenförmiges Bauteil ist, das teilweise durch die Befestigungsöffnung (103) in den Hohlraum eingesetzt ist, eine ringförmige Nut (311) an der Befestigungskomponente (31) um den Umfang der Befestigungskomponente (31) herum angeordnet ist und die Befestigungskomponente (31) in einer Einsetzrichtung in einen Einsetzabschnitt (312) und einen Befestigungsabschnitt (313) unterteilt, die luftdurchlässige Baumwolle (30) an dem Befestigungsabschnitt (313) angebracht ist und die Erfassungsbaugruppe einen Dichtungsring (32) umfasst, der in der ringförmigen Nut (311) eingebettet und so ausgebildet ist, dass er in Dichtungspassung mit dem Umfang der Befestigungsöffnung (103) ist, wenn der Befestigungsabschnitt (313) in den Hohlraum eingesetzt ist; und/oder
der Hohlraum mit einer darin angeordneten Trennwandanordnung versehen ist, die den Hohlraum in einen Lufteinlasshohlraum (11), einen Lüfterbefestigungshohlraum (12) und einen Luftauslasshohlraum (13) unterteilt, wobei der Lufteinlass (101) mit dem Lufteinlasshohlraum (11) in Verbindung steht, der Luftauslass (102) mit dem Luftauslasshohlraum (13) in Verbindung steht, der Lufteinlasshohlraum (11) mit dem Lüfterbefestigungshohlraum (12) über ein erstes, in der Trennwandanordnung ausgebildetes Durchgangsloch (141) in Verbindung steht, der Lüfterbefestigungshohlraum (12) mit dem Luftauslasshohlraum (13) über ein zweites, in der Trennwandanordnung ausgebildetes Durchgangsloch (151) in Verbindung steht, und die Befestigungsöffnung (103) so ausgebildet ist, dass sie mit dem Lufteinlasshohlraum (11) in Verbindung steht.

8. Schallschluckbox nach Anspruch 7, wobei die luftdurchlässige Baumwolle (30) plattenförmig ist, wobei
der Befestigungsabschnitt (313) so angeordnet ist, dass er durch die Befestigungsöffnung (103) in einer Richtung senkrecht zu einer Luftströmungsrichtung in dem Hohlraum in den Hohlraum eingesetzt werden kann, wobei der Befestigungsabschnitt (313) als ringförmiger Befestigungsrahmen ausgebildet ist und die luftdurchlässige Baumwolle (30) in dem Befestigungsrahmen eingebettet ist; oder
der Befestigungsabschnitt (313) so angeordnet ist, dass er durch die Befestigungsöffnung (103) in einer Richtung parallel zur Luftströmungsrichtung in dem Hohlraum in den Hohlraum eingesetzt werden kann, und eine obere Fläche des Befestigungsabschnitts (313) mit einer Befestigungsnut (315) zum Einbetten der luftdurchlässigen Baumwolle (30) darin versehen ist.

9. Schallschluckbox nach Anspruch 7, wobei der Hohlraum mit einer Führungsstruktur (111) zum Führen des Einführens des Befestigungsabschnitts (313) und zum Stützen des Befestigungsabschnitts (313) darin versehen ist.

10. Schallschluckbox nach einem der Ansprüche 1 bis 9, wobei das Gehäuse (10) ein Gehäuseoberteil (18) und ein Gehäuseunterteil (19) umfasst, die Schallschluckbox ein Dichtungselement (40) umfasst, das zwischen dem Gehäuseoberteil (18) und dem Gehäuseunterteil (19) angeordnet ist, und die Erfassungsbaugruppe an dem Gehäuseoberteil (18) oder dem Gehäuseunterteil (19) angebracht ist.

11. Therapeutische Ventilationsvorrichtung, umfassend die Schallschluckbox gemäß einem der Ansprüche 1 bis 10.

## Revendications

1. Boîte de réduction de bruit, comprenant une coque (10) et un ensemble de détection, dans laquelle l'intérieur de la coque (10) définit une cavité, la coque (10) est pourvue d'un orifice d'entrée d'air (101) et d'un orifice de sortie d'air (102) qui se trouvent en communication avec la cavité, et l'ensemble de détection est monté sur la coque (10) ; **caractérisée en ce que**
l'ensemble de détection est adapté pour détecter le degré auquel l'intérieur de la cavité est sale, de sorte que la boîte de réduction de bruit peut être nettoyée et entretenue à temps, dans laquelle l'ensemble de détection comprend un composant **d'adsorption** de poussière et le composant d'adsorption de poussière est adapté pour absorber les polluants dans l'air dans la cavité et pour révéler la couleur à différents degrés en fonction de la quantité des polluants absorbés.

2. Boîte de réduction de bruit selon la revendication **1,** dans laquelle le composant d'adsorption de poussière est une paroi transparente (20), qui est formée comme une partie de la coque (10).

3. Boîte de réduction de bruit selon la revendication **2,** dans laquelle **l'ensemble** de détection comprend une source de lumière (21), qui est agencée dans la cavité pour éclairer la paroi transparente (20).

4. Boîte de réduction de bruit selon la revendication **2,** dans laquelle la cavité est pourvue d'un ensemble de partitionnement à l'intérieur, qui divise la cavité en une cavité de prise d'air (11), une cavité de montage de ventilateur (12) et une cavité **d'évacuation** d'air (13), dans laquelle l'orifice d'entrée d'air (101) se trouve en communication avec la cavité de prise d'air (11), l'orifice de sortie d'air (102) se trouve en communication avec la cavité d'évacuation d'air (13), la cavité de prise d'air (11) se trouve en communication avec la cavité de montage de ventilateur (12) à travers un premier trou traversant (141) formé dans **l'ensemble** de partitionnement, la cavité de montage de ventilateur (12) se trouve en communication avec la cavité **d'évacuation** d'air (13) à travers un second trou traversant (151) formé dans l'ensemble de partitionnement, la source de lumière (21) est agencée dans la cavité de prise d'air (11), et la paroi transparente (20) est formée comme une partie de la coque (10) pour définir la cavité de prise **d'air** (11).

5. Boîte de réduction de bruit selon la revendication **1,** dans laquelle le composant d'adsorption de poussière est un coton perméable à l'air (30), qui est au moins partiellement agencé dans la cavité et qui peut être sorti de la cavité.

6. Boîte de réduction de bruit selon la revendication **5,** dans laquelle **l'ensemble** de détection comprend un composant de montage (31) pour monter le coton perméable à **l'air** (30), la coque (10) est pourvue d'un orifice de montage (103) en communication avec la cavité, et le composant de montage (31) est monté de manière amovible dans l'orifice de montage (103) de sorte que le coton perméable à l'air (30) est positionné dans la cavité.

7. Boîte de réduction de bruit selon la revendication **6,** dans laquelle
le composant de montage (31) est un composant en forme de plaque partiellement inséré dans la cavité à travers l'orifice de montage (103), une rainure annulaire (311) est agencée sur le composant de montage (31) autour de la périphérie du composant de montage (31) et divise le composant de montage (31) en une portion **d'insertion** (312) et une portion de montage (313) dans un sens d'insertion, le coton perméable **à l'air** (30) est monté sur la portion de montage (313), et l'ensemble de détection comprend un anneau d'étanchéité (32) enchâssé dans la rainure annulaire (311) et configuré se trouver en ajustement hermétique avec la périphérie de l'orifice de montage (103) lorsque la portion de montage (313) est insérée dans la cavité ; et/ou
la cavité est pourvue d'un ensemble de partitionnement à l'intérieur, qui divise la cavité en une cavité de prise d'air (11), une cavité de montage de ventilateur (12) et une cavité d'évacuation d'air (13), dans laquelle l'orifice d'entrée d'air (101) se trouve en communication avec la cavité de prise d'air (11), l'orifice de sortie d'air (102) se trouve en communication avec la cavité d'évacuation d'air (13), la cavité de prise d'air (11) se trouve en communication avec la cavité de montage de ventilateur (12) à travers un premier trou traversant (141) formé dans l'ensemble de partitionnement, la cavité de montage de ventilateur (12) se trouve en communication avec la cavité d'évacuation d'air (13) à travers un second trou traversant (151) formé dans l'ensemble de partitionnement, et l'orifice de montage (103) est configuré pour se trouver en communication avec la cavité de prise d'air (11).

8. Boîte de réduction de bruit selon la revendication 7, dans laquelle le coton perméable à l'air (30) est en forme de plaque, dans laquelle
la portion de montage (313) est agencée pour être insérée dans la cavité à travers l'orifice de montage (103) dans un sens perpendiculaire à un sens d'écoulement d'air dans la cavité, la portion de montage (313) est formée comme un cadre de montage annulaire, et le coton perméable à l'air (30) est enchâssé dans le cadre de montage ; ou
la portion de montage (313) est agencée pour être insérée dans la cavité à travers l'orifice de montage (103) dans un sens parallèle au sens de l'écoulement de l'air dans la cavité, et une surface supérieure de la portion de montage (313) est pourvue d'une rainure de montage (315) pour enchâsser à l'intérieur le coton perméable à l'air (30).

9. Boîte de réduction de bruit selon la revendication 7, dans laquelle la cavité est pourvue d'une structure de guidage (111) pour guider l'insertion de la portion de montage (313) et supporter la portion de montage (313) à l'intérieur.

10. Boîte de réduction de bruit selon l'une quelconque des revendications 1 à 9, dans laquelle la coque (10) comprend une coque supérieure (18) et une coque inférieure (19), la boîte de réduction de bruit comprend un élément d'étanchéité (40) en sandwich entre la coque supérieure (18) et la coque inférieure (19), et l'ensemble de détection est monté sur la coque supérieure (18) ou sur la coque inférieure (19).

11. Dispositif thérapeutique de ventilation, comprenant la boîte de réduction de bruit selon l'une quelconque des revendications 1 à 10.
